# EUROPEAN PATENT APPLICATION

(11) **EP 0 798 379 A2**
(43) Date of publication of application: **01.10.1997**
(21) Application number: 97302110.8
(22) Date of filing: 26.03.1997
(51) Int. Cl.: C12N 15/60, C12N 5/18, C12N 9/88, C07K 16/40, G01N 33/573, G01N 33/543

(54) **Human glutamic acid decarbocylase-expressing myeloma cell line**

(30) Priority: 29.03.1996 JP 76681/96
(71) Applicant: Tosoh Corporation, Shinnanyo-shi, Yamaguchi-ken, 746 (JP)
(72) Inventor: Matsuba, Takao, Atsugi-shi, Kanagawa (JP); Yasukawa, Kiyoshi, Kawasaki-shi, Kanagawa (JP)
(74) Representative: Kearney, Kevin David Nicholas

(57) **Abstract**

A method for producing a large quantity of glutamic acid decarboxylase, and a method for assaying anti-glutamic acid decarboxylase antibody are provided. For the production, a myeloma cell strain SPG14 which expresses recombinant glutamic acid decarboxylase is cultivated, and the recombinant glutamic acid decarboxylase is recovered. This glutamic acid decarboxylase is bonded to a water-insoluble carrier, and is brought into contact with an assay sample. Then, an anti-glutamic acid decarboxylase antibody in the assay sample bonded to the glutamic acid decarboxylase is detected selectively.

SEQUENCE LISTING

(1) GENERAL INFORMATION:

(i) APPLICANT:
(A) NAME: TOSOH CORPORATION
(B) STREET: 4560 KAISEI-CHO
(C) CITY: SHINNANYO-SHI
(D) STATE: YAMAGUCHI
(E) COUNTRY: JAPAN
(F) POSTAL CODE (ZIP): JAPAN

(ii) TITLE OF INVENTION: HUMAN GLUTAMIC ACID DECARBOXYLASE-EXPRESSING
MYELOMA CELL LINE

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 97302110.8
(vi) PRIOR APPLICATION DATA:
(A) APPLICATION NUMBER: JP 8-76681
(B) FILING DATE: 29-MAR-1996

(2) INFORMATION FOR SEQ ID NO: 1:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 1:

TTGTCGACAT GGCATCTCCG GGCTCTGGC 29

(2) INFORMATION FOR SEQ ID NO: 2:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 2:

GGGCGGCCGC TCTAGAAATC TTGTCCTAGG CGTTC 35

## Description

### Background of the Invention:

### Field of the Invention:

The present invention relates to a myeloma cell line which expresses a recombinant human glutamic acid decarboxylase, a process for producing the recombinant human glutamic acid decarboxylase, an assaying method of an anti-human glutamic acid decarboxylase antibody, and a reagent kit for assaying the anti-human glutamic acid decarboxylase antibody.

### Description of the Related Art:

The glutamic acid decarboxylase (hereinafter referred to as GAD) is an enzyme which synthesizes γ-aminobutyric acid, an inhibitory neurotransmitter, from glutamic acid. This enzyme is contained in brains and pancreatic insula Langerhansis. Recently, GAD was found to be a main autoantigen of insulin-dependent diabetes mellitus (IDDM) (Beakkeskov, et al.: Nature, 347, 151 (1990)). The anti-GAD autoantibody, which is contained in the blood of IDDM patients in high probability, is utilized as a marker in diagnosis and preperception of IDDM in clinic at present. A kit for radioimmunoassay (RIA) of IDDM is commercialized (RippAnti-GAD Hoechst (trade name), Hoechst Co.). However, the conventional assay method involves two problems.

One problem is necessity for using a radioactive substance. Obviously, enzyme immunoassay (EIA) using no radioactive substance can be conducted more readily than RIA using a radioactive substance. However, at a lower concentration of the components constituting the assaying system, sufficient sensitivity is not achievable by the EIA, so that the RIA is employed unavoidably. A GAD which will bond to an anti-GAD autoantibody is reported to be prepared from a swine brain (natural type GAD), or from recombinant GAD-expressing CHO cells (recombinant type GAD). However, the GAD productivity is low. For example, from one swine brain, GAD can presumably be prepared at a yield of as low as 20-100 µg. On the other hand, recombinant GAD was reported to be developed from Escherichia coli, or insect cells. However, the resulting GAD could not constitute an assaying system because of poor bonding to the anti-GAD autoantibody. More specifically, the recombinant GAD from Escherichia coli or insect cells, although the yield is high, is not convertible to GAD of the natural type structure even by refolding treatment, being less reactive to the anti-GAD autoantibody, disadvantageously. For satisfactory practice of EIA, GAD is required to be prepared by a simple method in an amount of at least about one milligram which is a necessary amount for reaction with an anti-GAD autoantibody in the serum of a patient.

Another problem is that the available amount of GAD is insufficient to be supported by a water-insoluble carrier. If GAD can be supported by a water-insoluble carrier, an anti-GAD autoantibody can be detected specifically by bringing the supported GAD into contact with a sample to be assayed to form an antigen-antibody complex, and subsequently removing non-bonded components. Such a method can be conducted by an automatic process, and can be incorporated as one assay item into an automatic immunoassay apparatus for measuring plural items. In a conventional assaying system, the anti-GAD autoantibody in a sample is assayed through steps of bringing an isotope-labeled GAD into contact with an assay sample, mixing it with a goat serum containing an anti-human immunoglobulin and with protein A bonded to a carrier resin and capable of bonding specifically to the antibody, separating the precipitate by centrifuge, and measuring radioactivity of the precipitate. This method cannot readily be automated. On the other hand, the bonding properties of anti-GAD autoantibody to GAD has not been investigated, and it is not known whether or not the anti-GAD autoantibody can be assayed by use of a recombinant GAD, in principle.

### Summary of the Invention:

An object of the present invention is to provide a simpler process for production of GAD in a milligram level.

Another object of the present invention is to provide a method for assaying an anti-GAD antibody without employing a radioactive substance.

Still another object of the present invention is to provide a method for detecting specifically an anti-GAD autoantibody by analyzing bond-formation properties between the anti-GAD autoantibody and GAD, bringing GAD bonded to a water-insoluble carrier in contact with an assay sample, separating the anti-GAD autoantibody bonded through GAD to the water insoluble carrier from non-bonded components, detecting specifically the anti-GAD autoantibody bonded through the GAD to the water-insoluble carrier.

To achieve the above objects, the inventors of the present invention, after comprehensive studies on expression of GAD, established a myeloma cell line which expresses recombinant GAD and can be cultivated effectively to produce and recover GAD. Further, the inventors obtained purified GAD by cultivating the cell line, and isolating and purifying the GAD. Furthermore, the inventors accomplished an immunoassay method of anti-GAD antibody by use of the purified GAD.

According to an aspect of the present invention, there is provided a myeloma cell line which expresses a recombinant human glutamic acid decarboxylase.

According to another aspect of the present invention, there is provided a process for producing a recombinant human glutamic acid decarboxylase, comprising cultivating the above myeloma cell line, and recovering the recombinant human glutamic acid decarboxylase.

According to still another aspect of the present invention, there is provideda method of assaying an anti-human glutamic acid decarboxylase antibody, comprising bringing the human glutamic acid decarboxylase produced by the above process into contact with an assay sample, and detecting anti-human glutamic acid decarboxylase antibody bonded with the human glutamic acid decarboxylase.

According to a still further aspect of the present invention, there is provided a reagent kit for the method of assay, containing the human glutamic acid decarboxylase produced by the above production process.

According to a still further aspect of the present invention, there is provided a process for producing an anti-human glutamic acid decarboxylase antibody, comprising immunizing an animal by the human glutamic acid decarboxylase produced by the above production process.

According to a still further aspect of the present invention, there is provided a method of assaying an anti-human glutamic acid decarboxylase antibody, comprising bonding a human glutamic acid decarboxylase onto a water-insoluble carrier, bringing the human glutamic acid decarbozylase into contact with an assay sample, separating the anti-human glutamic acid decarboxylase antibody bonded to the human glutamic acid decarboxylase from non-bonded components, and detecting specifically the anti-human glutamic acid decarboxylase antibody bonded to the human glutamic acid decarboxylase.

According a still further aspect of the present invention, there is provided a reagent kit for the above method of assay, comprising a human glutamic acid decarboxylase bonded to a water-insoluble carrier.

### Brief Description of the Drawings:

Fig. 1 illustrates the structure of a GAD-expressing vector BCMGS-GAD.

Fig. 2 shows the results of western blotting conducted in Example 3.

Fig. 3 shows the result of SDS-PAGE of a purified GAD.

Fig. 4 shows a calibration curve prepared by use of purified GAD65.

Fig. 5 shows dependence of the radioactivity of the precipitate on the concentration of the SPG14 cellular extract in Example 6.

Fig. 6 shows dependency of the absorbance on the standard serum concentration in Example 7.

Fig. 7 shows the absorbance of the serum of the IDDM patients and the serum of the healthy persons in Example 8.

Fig. 8 shows the absorbance of the serum of the IDDM patients and the healthy persons in Example 9.

Fig. 9 shows correlation between the measurement results of the IDDM patient serum measured by a sandwich enzyme immunoassay and the measurement result by RippAnti-GAD Hoechst in Example 9.

### Detailed Description of the Preferred Embodiment:

The present invention is described below in detail.

### 1. Recombinant GAD-Expressing Myeloma Cell line:

The myeloma cell line which expresses recombinant human GAD of the present invention is prepared by introducing an expression vector into a myeloma cell line by usual genetic engineering technique as below.

The reproducible expression vector which expresses a DNA sequence for coding the GAD is employed in the present invention. This expression vector is not specially limited, but is required to have the known DNA sequence for producing GAD, a DNA sequence for expressing the above DNA sequence, and a replication origin for replicating a vector DNA, and to be capable of transforming the host. For the DNA sequence for expressing the intended DNA, a promoter system is important. The promoter system includes SV40 promoter systems, and cytomegalovirus promoter systems, and is selected in consideration of the host.

The GAD includes molecular species of GAD65 and GAD67, both being useful. For detecting the anti-GAD antibody accompanied by IDDM, the DNA sequence is preferred which is capable of coding the human GAD65 as the GAD-coding DNA sequence in the present invention. The DNA sequence can be prepared according to a known literature (e.g., Karlsen, A.E., et al: Proc. Natl. Acad. Sci. USA: 88 8337 (1991)).

The myeloma cell line applicable in the present invention includes not only the mouse myeloma cell line SP2/0 (non-immunoglobulin secreting mouse myeloma, CRL1581; ATCC) used in Examples but also P3U1, NS1, and the like.

The transformation of the myeloma cell line by an expression vector may be conducted by a conventional method such as electroporation, and the method is not limited specially.

The myeloma cell line can be cultivated by a conventional method. Specifically, the cultivation is conducted by standing cultivation with a usual cultivation tools, or is conducted by a large-scale cultivation process with a suitable cell cultivation apparatus. The cell cultivation apparatus is exemplified by hollowfiber type apparatuses and tank type apparatuses. Of these, tank type apparatuses are preferred because of needlessness of removal of the cells from the carrier, and other reasons.

The GAD-coding DNA sequence in the myeloma cell is expressed by the action of the promoter system, whereby myeloma cells which expresses GAD is produced. One method for preparing GAD from the myeloma cells comprises adding a buffer solution to the cells, homogenizing the cells, centrifuging the homogenate to recover the supernatant liquid, subjecting the recovered supernatant to affinity chromatography employing anti-GAD antibody to purify the GAD. The antibody employed for the chromatography includes GAD1 antibody shown in Examples described later. In such a manner, the myelomas SPG12, SPG14, SPG19, SPG111, SPG35, SPG311, SPG46, SPG59, SPG68, SPG610, SPG72, SPG73, SPG713, SPG86, SPG87, and SPG88 which express recombinant human GAD are obtained in the present invention. Of these, SPG14 exhibited the highest productivity.

2. Assay Method for Anti-GAD Antibody: comprising bringing GAD produced by GAD-expressing myeloma cell line into contact with assay sample, and detecting anti-GAD antibody bonded to GAD:

In this assay method, the anti-GAD antibody is assayed by bringing GAD produced by a GAD-expressing myeloma cell line into contact with an assay sample, and detecting an anti-GAD antibody bonded to the GAD. In particular, the physiological concentration of an anti-GAD autoantibody in human serum can be assayed precisely and rapidly.

For example, RippAnti-GAD Hoechst (produced by Hoechst Co.), a commercially available kit, is used for assay of an anti-GAD autoantibody: the assay comprising steps of bringing isotope-labeled GAD into contact with an assay sample, mixing it with goat serum containing an anti-human immunoglobulin and with protein A bonded to a carrier resin and capable of bonding specifically to the antibody, separating the precipitate by centrifuge, and measuring radioactivity of the precipitate. In such an assay method, the recombinant GAD developed in the myeloma cell line of the present invention can be used as the GAD to be labeled.

3. Assay Method of Anti-GAD Antibody: comprising bonding GAD with water-insoluble carrier, bringing bonded GAD into contact with assay sample, separating anti-GAD antibody bonded to GAD from non-bonded components, detecting specifically the anti-GAD antibody bonded to GAD:

In this assay method, anti-GAD antibody in an assay sample is allowed to bond to the GAD bonded to a water-insoluble carrier, and the bonded anti-GAD antibody is assayed. In particular, the concentration of anti-GAD autoantibody in human serum can be assayed.

The applicable water-insoluble carrier includes plate-shaped materials such as microtiter plates, beads of a plastic material such as polystyrene and polypropylene, and beads of an inorganic material such as metals and ceramics.

The immobilization of the GAD on the water-insoluble carrier can be conducted by direct bonding of GAD, or indirect bonding of GAD with interposition of another substance. In a direct bonding method, the GAD at a concentration of 10 µg/mL in terms of protein, for example, is placed in wells of a plate in a suitable amount (e.g., about 100 µL), and is left standing overnight.

The immobilization of the GAD with interposition of a third substance can be conducted by use of a bridging agent or an anti-GAD antibody. In a specific example, an anti-GAD antibody solution in a suitable concentration (e.g., 2 µg/mL) in a PBS solution is placed in wells of a plate in a suitable amount (e.g. about 100 µL), and is left standing overnight, and further GAD at a concentration, for example, of 10 µg/mL in terms of protein is added to the plate wells in a suitable amount (e.g., about 100 µL), and is left standing overnight.

The anti-GAD antibodv bonded through GAD to the water-insoluble carrier can be assayed by a sandwich method, a competitive method, or a like method. In a sandwich method, a labeled anti-human immunoglobulin antibody is employed, and this labeled anti-human immunoglobulin antibody is allowed to bond specifically to an anti-GAD autoantibody bonded through GAD to a solid carrier, and the label is detected. On the other hand, in a competitive method, a labeled anti-GAD antibody is employed, and the labeled anti-GAD antibody added and an anti-GAD antibody in the sample compete with each other for bonding to GAD.

The labeling substance applicable in the present invention includes conventional radioactive substances, fluorescent substances, luminescent substances, enzymes such as alkaline phosphatase and horseradish peroxidase, and biotin. In view of ease of handling, enzymes such as alkaline phosphatase and horseradish peroxidase are particularly preferred.

### 4. Reagent Kit:

The reagent kit provided by the present invention is used for assay of the anti-GAD antibody of the present invention. This kit preferably contains the recombinant GAD produced by cultivation of the GAD-expressing myeloma cell line as described above. However, GAD prepared by a conventional method may be used depending on the assay method. The kit may contain additionally other reagents necessary for a sandwich method or a competitive method.

### 5. Method for Preparing Anti-GAD Antibody:

The method for preparing anti-GAD antibody of the present invention is characterized in that an animal species other than humans is immunized by employing, as the immunogen, GAD produced from the GAD-expressing myeloma cell line of the present invention.

For production of a monoclonal antibody, a mouse is used as the immunized animal, and for production of a polyclonal antibody a rabbit is used as the immunized animal, but not limited thereto.

The produced anti-GAD antibody is applicable in the immunological assay of anti-GAD autoantibody as described above.

The recombinant GAD, in particular the recombinant human GAD65, which is effective for diagnosis of diabetes can be produced in a quantity as large as milligrams according to the present invention. Various effects can be achieved by the expression of the GAD-coding DNA sequence in myeloma cells in the present invention as below. The GAD can be produced in a larger quantity. The myeloma cells, which is not adherent cells, can be cultivated relatively easily by suspension cultivation with a simpler equipment without special skill, in a larger amount than adherent cells. Moreover, the GAD prepared by the myeloma cells has inherently the same structure as the natural type GAD, so that refolding thereof is not necessary, and the GAD reactive to the anti-GAD antibody can be produced steadily.

The anti-GAD autoantibody in the serum of a diabetic patient is capable of bonding to the GAD supported on a water-insoluble solid carrier to be finally detected by a labeled human immunoglobulin antibody according to the present invention. Therefore, in anti-GAD autoantibody assay, the so-called BF separation (operation for separating the GAD bonded to the anti-GAD autoantibody and non-bonded GAD) is practicable, which makes feasible a sandwich assay method, advantageously.

The above two inventions can be employed in combination effectively.

The recombinant GAD prepared in a large quantity according to the present invention is applicable to various fundamental researches, such as investigation of the steric structure of GAD, investigation of the state of the bonding between GAD and anti-GAD autoantibody, establishment of a B lymphocyte for producing anti-GAD autoantibody, establishment of a GAD-reactive T lymphocyte, and so forth. The results of these investigation are expected to be effectively utilized for development of novel remedies for diabetes.

The present invention is described in more detail by reference to examples without limiting the invention.

### Example 1: Preparation of Human GAD65 Gene:

From the cDNA library of human pancreas (Clone Tech Co.), oligonucleotides of Sequence No. 1 and Sequence No. 2 (see Sequence Table below) were employed for amplification of the gene of human GAD65 by use of a commercial kit (PCR Amplification Kit, Takara Shuzo K.K.). From the resulting PCR reactant, human GAD65 gene was cloned by use of a commercial kit (TA Cloning Kit, Invitrogen Co.).

### Example 2: Construction of Human GAD65-Expressing Plasmid:

A recombinant GAD65-expressing vector was constructed from the GAD65 gene prepared in Example 1 as the starting material.

The vector prepared in Example 1 was cut out by use of SalI and NotI. The obtained fragment (about 1.8k base pairs) was introduced into a commercial vector (pBluescript, Toyobo K.K.), and then cut out by use of XhoI and NotI. The obtained fragment was inserted into an animal cell-expressing plasmid, BCMGSneo (Eur. J. Immunol.: 18, 97-104 (1988); and J. Exp. Med.: 169, 13-25 (1989)) to obtain a human GAD65-expressing plasmid BCMGS-GAD.

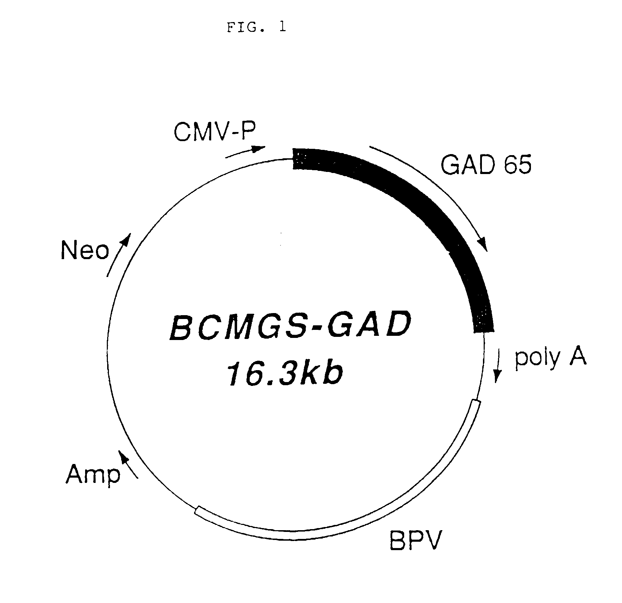

Fig. 1 shows the structure of the vector. In Fig. 1, CMV-P indicates a cytomegalovirus promotor, GAD indicates the DNA sequence for coding a human glutamic acid decarboxylase 65, BPV indicates the sequence for a bovine papilloma virus, Amp indicates the sequence for a β-lactamase, poly A indicates a polyadenylate attachment signal, and Neo indicates a neomycine-resistant gene.

### Example 3: Establishement of Human GAD65-Expressing Myeloma Cell Strain:

The BCMGS-GAD (10 µg) obtained in Example 2 was introduced into 2x10⁷ cells of SP2/0 (immunoglobulin non-secreting mouse myeloma, CRL1581; ATCC) by electroporation method (Gene Pluser, 900 V, 25 µF; BIORAD Co.). The cells were left standing on ice for 10 minutes. Then the cells were collected, and cultivated on an E-RDF medium (containing 10% FCS) for 24 hours. Thereafter, cells were cultivated on a selective medium (E-RDF medium, containing 10% FCS and 600 µg/mL of G418) for one day. Then the culture was subjected to monocloning by limiting dilution to obtain 16 clones.

The respective clones were analyzed for GAD65 expression by the western blot technique. Specifically, the cellular extract of the clones was subjected to separation by 10% acrylamide gel electrophoresis, and the separated fractions were transferred onto a nitrocellulose film. The film having the transferred fractions was allowed to react with an anti-GAD monoclonal antibody (GAD6: Developmental Studies Hybridoma Bank), and the antibody bonded to the film was allowed to react with an enzyme-labeled anti-mouse immunoglobulin. Then, color was developed by use of a commercial kit (ECL-Detection Kit, Amersham).

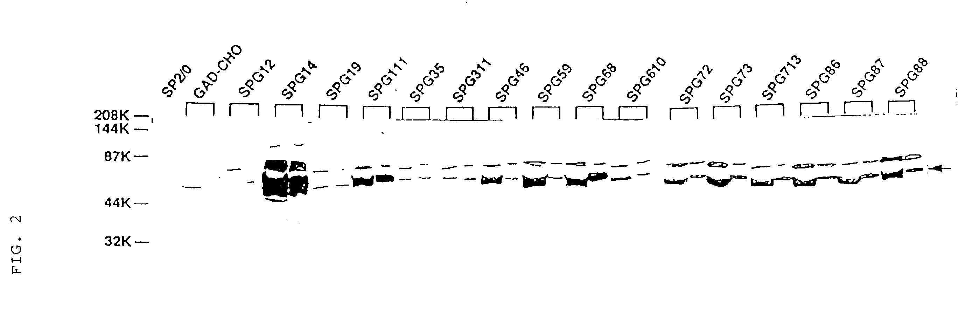

Fig. 2 shows the result. In Fig. 2, SP2/0 indicates the host cells, GAD-CHO indicates the known GAD-expressing CHO cells (Nippon Tonyobyo Gakkai (Japan Diabetes Society) 1995), and SPG12, SPG14, SPG19, SPG111, SPG35, SPG311, SPG46, SPG59, SPG68, SPG610, SPG72, SPG73, SPG713, SPG86, SPG87, and SPG88 indicate respectively the clone numbers. The quantity of the cellular extract for the right-hand clone line was 1/4 times as that for the lefthand clone line. In Fig. 2, the arrow mark indicates GAD65. The clone of the largest expression was established as a GAD65-developing myeloma cell line (SPG14).

### Example 4: Mass Cultivation of Human GAD65-developing Myeloma Cell line:

Mass cultivation of the SPG 14 was conducted by means of a tank type mass cultivation apparatus (Axellon, Kirin Brewery Co., Ltd.). 3×10⁸ SPG14 cells were inoculated to 3 liters of E-RDF medium (containing 10% FCS, and 600 µg/mL of G418; 1×10⁵ cells/mL). Cultivation was conducted for three days, whereby the cells increased to 1×10⁶ cells/mL. Then perfusion of the medium was started, and the cultivation was continued for three more days with the medium perfusion to increase the cell number to 1.6×10⁷ cells/mL. During the cultivation, the cultivation conditions were controlled at a temperature of 37°C, at a pH of 7.0, and at a dissolved oxygen content of 5 ppm.

After completion of the above cultivation, the cells were collected by centrifuge to recover 4.8×10¹⁰ cells from the 3-liter cultivation tank. The cells were rinsed once with PBS, and freezed and preserved at a temperature of -80°C.

### Example 5: Purification of Recombinant Human GAD65:

A GAD1-binding antibody column was prepared by use of 1 mL of HiTrap NHS-activated (Pharmacia Co.) and 10 mg of anti-human GAD antibody GAD1 (HB184; ATCC) according to the annexed protocol.

To the cells (8.4×10⁸ cells) corresponding to 1/50 portion of the cells obtained by the cultivation in Example 4, was homogenized with 5 mL of a lysing buffer solution (25mM potassium phosphate buffer solution (ph: 7.0) containing 0.2mM pyridoxal phosphate (PLP), 1mM 2-aminoethylisothiouronium bromide (AET) and 1mM benzamidine hydrochloride) by means of a teflon homogenizer. The resulting homogenate was centrifuged to obtain a supernatant liquid as the SPG14 cellular extract.

The SPG14 cellular extract was loaded into an antibody column equilibrated with an equilibrating buffer solution (50mM potassium phosphate buffer solution (pH: 7.2) containing 0.2mM PLP, and 1mM AET). The column was washed with 5 mL of the same buffer solution, and elution was conducted by use of an elution buffer solution (50mM potassium phosphate buffer solution (pH: 11.0) containing 0.2mM PLP, 1mM AET, 20mM glutamic acid, and 10mM diethanolamine). The eluted fraction was neutralized with 0.1N HCl. The above operation was repeated several times, and the obtained elution fractions were combined and concentrated by use of a ultrafiltration membrane of fraction molecular weight of 10,000.

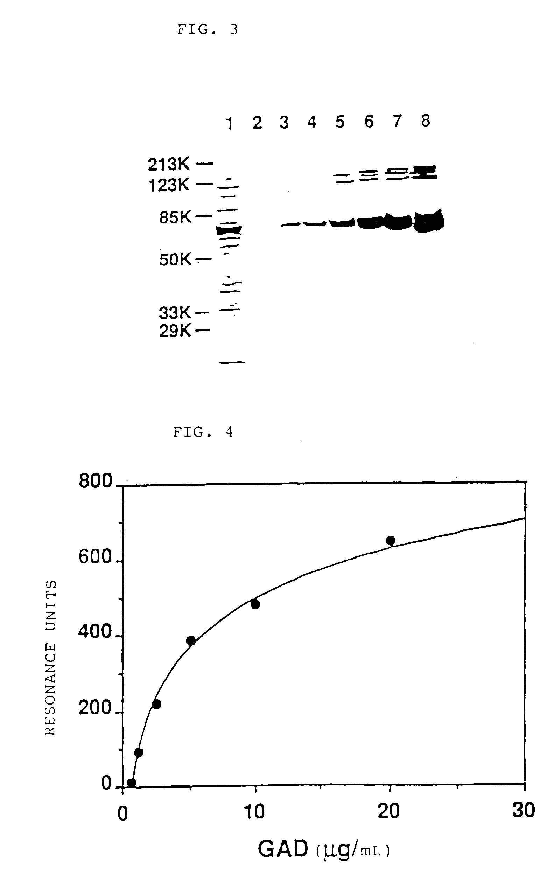

Fig. 3 shows the electrophoresis pattern of the condensed sample. Lane 1 shows the electrophoresis result of condensed sample. Lanes of from 8 to 2 show the electrophoresis results of a standard protein (BSA) at the concentrations of 1, 2⁻¹, 2⁻², 2⁻³, 2⁻⁴, 2⁻⁵, and 2⁻⁶ mg/mL respectively. The results show that the recombinant human GAD65 of Lane 1 was sufficiently purified. From the electrophoresis result, the concentration of the purified GAD65 was decided by considering that the recombinant human GAD65 on Lane 1 was in the same amount as the BSA of Lane 6.

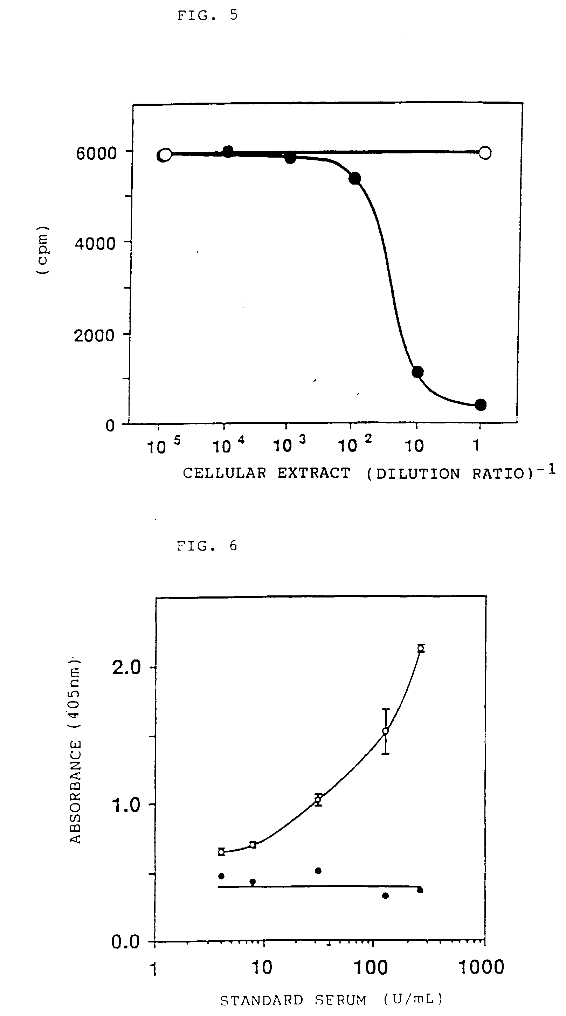

The concentration of GAD contained in the cellular extract liquid was measured with BIAcore (Pharmacia Co.), a surface plasma resonance measurement apparatus. Fig. 4 shows the result, in which the resonance units measured at various GAD concentrations with the BlAcore are plotted as a function of the GAD concentration.

The cells (8.4×10⁸ cells) corresponding to 1/50 portion of the cells obtained by the cultivation in Example 4 was homogenized with 5 mL of the aforementioned lysing buffer solution. The resulting homogenate was centrifuged to obtain Supernatant 1, and a precipitate. The precipitate was homogenized with 5 mL of the lysing buffer solution, and the homogenate was centrifuged to obtain Supernatant 2. The second precipitate was further homogenated with 5mL of the lysing buffer solution to obtain Supernatant 3. As the result, Supernatant 1, Supernatant 2, and Supernatant 3 were found to contain respectively 145.5 µg, 39 µg, and 13.5 µg of GAD65. Consequently, cells (8.4×10⁸ cells) corresponding to 1/50 portion of the cells obtained one batch of cultivation contained 200 µg of GAD65 (145.5 µg + 39 µg + 13.5 µg). Therefore, the one cultivation batch produced 10 mg in total of GAD65 (200 µg × 50), which shows the remarkably high productivity of the production process of the present invention.

### Example 6: Bonding Property of Recombinant Human GAD65 to Anti-GAD Autoantibody in Patient Serum:

To a serum containing an anti-GAD autoantibody of 125 U/mL as measured by use of a kit of RippAnti-GAD Hoechst (produced by Hoechst Co.), was added control cells (SP2/0), or a SPG14 cellular extract at various concentrations. The resulting mixture was further mixed with ¹²⁵I-GAD supplied by the RippAnti-GAD Hoechst to precipitate immunoglobulin. The radioactivity of the precipitate was measured. The cellular extract was prepared in the same manner as in Example 5. Fig. 5 shows the results. The measured radioactivities are plotted as a function of the dilution ratio of the coexisting cellular extract. In Fig. 5, the black circles show the results for the SPG14 cellular extract, and the white circles show the results for the SP2/0 cellular extract. Fig. 5 shows that the SPG14 cellular extract inhibited the bond formation between the anti-GAD autoantibody of a patient serum and the swine GAD of the used kit. In other words, the recombinant human GAD65 will bond to the anti-GAD autoantibody of a patient serum.

### Example 7: Sandwich Enzyme Immunoassay of Anti-GAD

### Autoantibody:

A calibration curve was prepared for sandwich enzyme immunoassay of an anti-GAD autoantibody by using purified GAD.

The purified GAD obtained in Example 5 was diluted to a concentration of 10 µg/mL, and 100 µL portions thereof were immobilized on a microtiter plate. Thereto, were added respectively 100 µL portions of 10-fold diluted fractions of the standard serum of 4, 8, 32, 125, and 256 U/mL of the RippAnti-GAD Hoechst kit to allow the anti-GAD autoantibody in the standard serum to react with the immobilized GAD on the plate. The anti-GAD autoantibody bonded to the GAD was allowed to react with anti-human IgG labeled with an enzyme, and color was developed with a coloring reagent.

Fig. 6 shows the results. Fig. 6 shows that the color development was significantly more intense in the wells holding the immobilized GAD reacted with the standard serum (white circles) than in the wells having no immobilized GAD (black circles), and that the color development was more intense at the higher antibody titer.

### Example 8: Sandwich Enzyme Immunoassay:

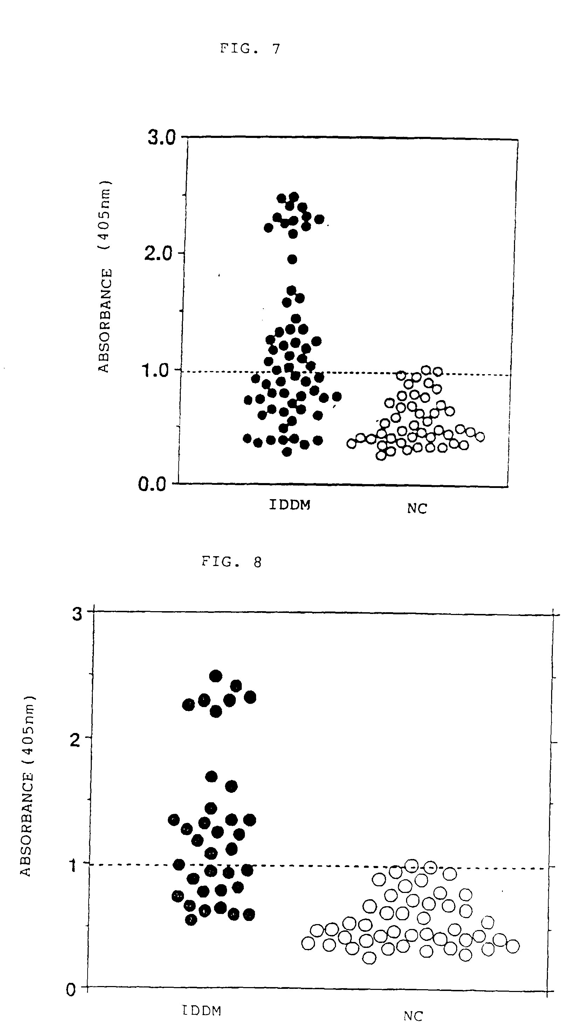

The anti-GAD autoantibody in the serums of IDDM patients (63 cases) and of healthy persons (45 cases) was assayed by the sandwich enzyme immunoassay method for anti-GAD autoantibody as conducted in Example 7. Fig. 7 shows the results. In Fig. 7, IDDM means insulin-dependent diabetes mellitus patients, and NC means healthy persons, and the dotted line denotes the value of "average + 2SD" of the serums of the healthy persons. As shown in Fig. 7, the measured values of the IDDM patient serums are significantly higher than that of the healthy persons. Thus, the anti-GAD autoantibody of a patient serum can be assayed by the sandwich enzyme immunoassay.

### Example 9:

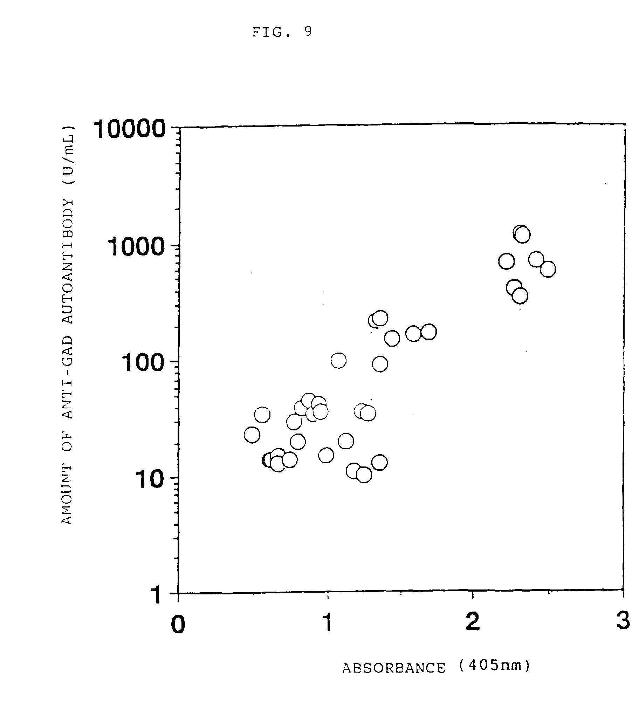

The anti-GAD autoantibody in the serums of IDDM patients (35 cases) and of healthy persons (45 cases) was assayed by the sandwich enzyme immunoassay method for anti-GAD autoantibody as conducted in Example 7. Fig. 8 shows the results. In Fig. 8, IDDM means insulin-dependent diabetes mellitus patients, and NC means healthy persons; and the dotted line denotes the value of "average + 2SD" of the serums of the healthy persons. As shown in Fig. 8, the measured values of the 21 IDDM patient serums corresponding to 60% of 35 patients are higher than the "average + 2SD" value of the serum of healthy persons. Thus, the anti-GAD autoantibody of a patient serum can be assayed by the sandwich enzyme immunoassay.

Further, the samples of the above 35 IDDM patient serums were subjected to assay *of* the anti-GAD antibody titer with the RippAnti-GAD Hoechst (produced by Hoechst Co.), and the measured data were compared with the above measured values according to the sandwich enzyme immunoassay. Fig. 9 shows the results. In Fig. 9, the ordinate shows the anti-GAD autoantibody titer (U/mL) measured by the RippAnti-GAD Hoechst kit (produced by Hoechst Co.), and the abscissa shows the absorbance at 405 nm measured by the sandwich enzyme immunoassay. Thus, high correlation was confirmed between the sandwich enzyme immunoassay conducted in Example 7 and the RippAnti-GAD Hoechst method for the anti-GAD autoantibody. In other words, the sandwich enzyme immunoassay exhibits sufficiently high sensitivity in identification of IDDM.

Sequence Table
Sequence No.: 1
Sequence length: 29
Sequence type: Nucleic acid
Strand number: Single strand
Topology: Linear

Sequence:            TTGTCGACAT GGCATCTCCG GGCTCTGGC  29.

Sequence No.:        2

Sequence length:     35

Sequence type:       Nucleic acid

Strand number:       Single strand

Topology:            Linear

Sequence:       GGGCGGCCGC TCTAGAAATC TTGTCCTAGG CGTTC   35.

## Claims

1. A myeloma cell line, expressing a recombinant human glutamic acid decarboxylase.

2. The myeloma cell line according to claim 1, wherein the cell line is an SPG14 cell line.

3. A process for producing a recombinant human glutamic acid decarboxylase, comprising cultivating the myeloma cell line set forth in claim 1, and recovering the recombinant human glutamic acid decarboxylase.

4. The process according to claim 3 wherein the myeloma cell line is an SPG14 cell line.

5. A method of assaying an anti-human glutamic acid decarboxylase antibody, comprising bringing the human glutamic acid decarboxylase produced by the process set forth in claim 3 or 4 into contact with an assay sample, and detecting anti-human glutamic acid decarboxylase antibody bonded with the human glutamic acid decarboxylase.

6. A method of assaying an anti-human glutamic acid decarboxylase antibody, comprising bonding a human glutamic acid decarboxylase onto a water-insoluble carrier, bringing the human glutamic acid decarboxylase into contact with an assay sample, separating the anti-human glutamic acid decarboxylase antibody bonded to the human glutamic acid decarboxylase from non-bonded components, and detecting specifically the anti-human glutamic acid decarboxylase antibody bonded to the human glutamic acid decarboxylase.

7. A reagent kit for the method of assay set forth in claim 5, containing the human glutamic acid decarboxylase produced by the production process set forth in claim 3 or 4.

8. A reagent kit for the method of assay set forth in claim 6, comprising a human glutamic acid decarboxylase bonded to a water-insoluble carrier.

9. A process for producing an anti-human glutamic acid decarboxylase antibody, comprising immunizing an animal by the human glutamic acid decarboxylase produced by the production process set forth in claim 3 or 4.
